# EUROPEAN PATENT SPECIFICATION

(11) **EP 1 482 835 B1**
(45) Date of publication and mention of the grant of the patent: **12.09.2012**
(21) Application number: 03743435.4
(22) Date of filing: 06.03.2003
(51) Int. Cl.: A61B 5/16

(54) **ANALOG SCALE FOR MEASURING PAIN**
ANALOGE SKALA ZUR MESSUNG VON SCHMERZEN
ECHELLE ANALOGIQUE POUR MESURER LA DOULEUR

(30) Priority: 06.03.2002 EP 02290551
(43) Date of publication of application: 08.12.2004
(73) Proprietor: EURO-CELTIQUE S.A., 1653 Luxembourg (LU)
(72) Inventor: MOREAU, Isabelle, F-78150 Le Chesnay (FR); FAUCHE, Christine, F-92600 Asnières (FR); ABRIC, André, F-92100 Boulogne (FR)
(74) Representative: Williams, Gareth Owen
(86) International application number: PCT/GB2003/000938
(87) International publication number: WO 2003/073937

(56) References cited:
- US-A- 4 250 891
- US-A- 4 582 251
- US-A- 4 614 042
- US-A- 5 018 526

## Description

### FIELD OF THE INVENTION

The present invention relates generally to a pain measuring device.

### BACKGROUND OF THE INVENTION

In diagnosing and treading patients suffering from varying levels of pain, the healthcare providers must rely on the patient's subjective description of the pain level being experienced. This presents a significant problem to healthcare providers because the subjective descriptions are qualitative and the subjective descriptions each patient chooses to use are not necessarily standardized. For example, some patients use the same description for the pain they are experiencing even though the pain may have increased or decreased.

Some alleged solutions to the above-described problem have been described in United Kingdom Patent No. 2,049,431 and United States Patent No. 6,258,042. The disclosures of these two references are hereby incorporated by reference. United Kingdom Patent No. 2,049,431 discloses a device generally known in the healthcare industry as a visual analog scale VAS). The device is a hand-held panel having a first scale for patients provided on one side and a corresponding second scale for the healthcare providers provided on the other side. The patient's pain scale is a straight line with "no pain" indicated at one end and "intense pain" indicated at the other end of the line, and a movable slider that a patient would use to indicate a position on the straight line pain scale which corresponds to the pain level felt by the patient. The healthcare provider's pain scale is supplemented with graduated intervals and numbers, such as 0 through 10 in order to translate the pain level indicated by the patient's positioning of the slider to a corresponding value between 0 to 10 shown on the healthcare provider's pain scale. This allows the healthcare provider to quantity the patient's pain levels.

United States Patent No. 6,258,042 discloses a VAS device similar to the device of the United Kingdom patent reference with an added feature of verbal pain descriptors provided on the provider's side of the device. A different verbal descriptor corresponding to each of the eleven numerical pain descriptors 0 through 10 are provided.

An inherent limitation of the prior art VAS devices is the fact that the patient's ability to see the device may often affect the pain level indicated by the patient's positioning of the slider. Hence, the prior art VAS devices are often difficult to use and/or inaccurate for visually-impaired patients. Accordingly there is a need for a pain-measuring device which may be used more easily and accurately by visually-impaired patients, particularly in view of the number of elderly pain, management patients having impaired vision.

US 4,250,891 discloses a device for measuring fingertip sensitivity comprising a housing bar and a feeler bar that is horizontally slideable within the housing. The device is used to determine depth-sense perception, spread perception or 2-point discrimination.

US 4,614,042 discloses a ruler for use in measuring small distances. The device Is designed for use by the visually impaired or the blind.

### SUMMARY OF THE INVENTION

The present invention is directed towards an analog scale and a method for measuring the level of pain that a person is experiencing. More particularly, the analog scale of the invention is configured to be used more easily and accurately by visually-impaired patients, as well as by patients with no visual impairment.

The analog scale of the invention is a handheld panel-like device having two sides. One side of the panel, the patient's side, bears a patient's pain scale that is configured and dimensioned in such a manner as to exhibit a three-dimensional structure so that a visually-impaired patient can sense level of pain indicated by the scale by touching the scale. For example, in a preferred embodiment of the invention, the patient's pain scale is provided in a relief on the surface of the analog scale (*i.e*., raised on the surface). One end of the patient's pain scale represents no pain and the other end represents maximum or constant severe pain. In a preferred embodiment of the invention, the patient's pain scale further comprises one or more dimensions which vary from one end of the scale to the other to further enhance the visually-impaired patient's ability to sense the level of pain indicated by the scale by touching the scale.

The other side of the panel, the healthcare provider's side, bears a healthcare provider's pain scale that is graduated and marked with at least numbers, such as 0 through 10 at various intervals. The numbers 0 to 10 represent increasing levels of pain so that the end marked with the number 0 corresponds to the "no pain" end of the patient's pain scale and the end marked with the highest number corresponds to the maximum pain end of the patient's pain scale.

The analog scale is provided with a slider slidably mounted on the panel. The slider is configured to be movable between the two ends of the pain scales and simultaneously points to corresponding points along the patient's side of the pain scale and the provider's side of the pain scale. In other words, when the slider is pointing to "no pain" on the patient's pain scale, it is also pointing to the corresponding number "0" and/or the words "no pain" on the healthcare provider's side of the pain scale. Similarly, when the slider is pointing to "maximum pain" on the patient's side of the scale, it is also pointing to the corresponding number, such as "10" and/or words such as "maximum pain" on the healthcare provider's side of the pain scale. The slider is provided with one or more indicators or markings, such as a hairline, so that one can determine which points along the two sides of the pain scale the slider is pointing to.

In use, the analog pain scale is presented so that the patient uses the slider to indicate the level of pain that the patient is experiencing by positioning the slider at an appropriate point along the patient's pain scale. This qualitative indication of the patient's pain level on the scale that ranges from "no pain" to "maximum pain" is then translated into a quantitative and/or qualitative description on the scale on the provider's side of the pain scale which includes corresponding numbers and/or words. Because the patient's pain scale is provided on the analog scale in a relief, a visually-impaired patient is able to understand the relative level of pain being indicated by their positioning of the slider more easily and accurately. In other words, by touching the pain scale, a visually impaired person may determine at which point along the scale to position the slider in order to represent their pain level.

### BRIEF DESCRIPTION OF THE DRAWINGS

The features and advantages of the present invention will now be described in detail with the aid of the following drawings in which like elements are labelled similarly and in which:
**Figure 1** is a perspective view of the patient's side of an embodiment of the analog scale of the present invention;
**Figure 2** is a perspective view of the provider's side of the analog scale of **Figure 1**;
**Figure 3** is a perspective view of the embodiment of the patient's side of a slider on an analog scale of the present invention;
**Figure 4** is a perspective view of the patient's side of an embodiment of the analog scale of the present invention; and
**Figure 5** is a perspective view of the provider's side of the analog scale of **Figure 4**.

### DETAILED DESCRIPTION OF THE INVENTION

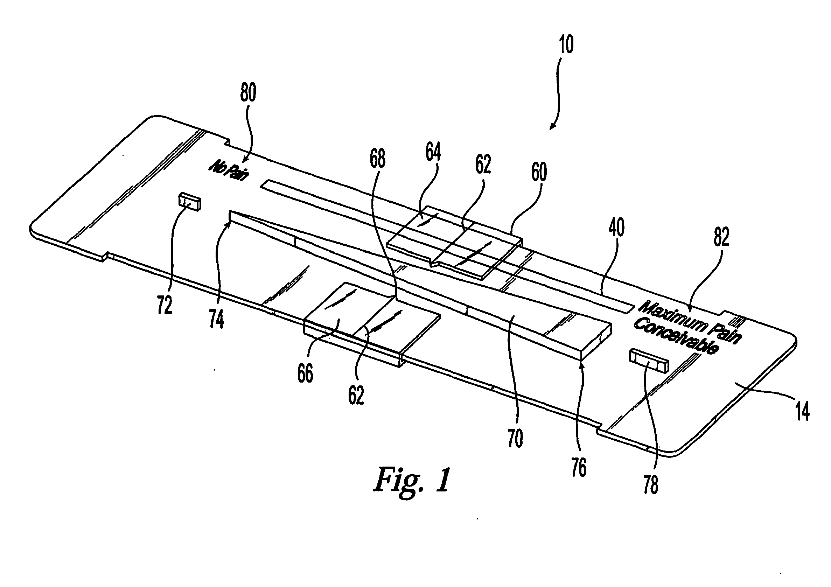

**Figure 1** illustrates the patient's side **(14)** of an analog scale **(10)** according to one preferred embodiment of the invention. On the patient's side **(14)**, a patient's pain scale **(70)** is provided. The patient's pain scale **(70)** is provided in a relief so that, by touching the pain scale, a visually-impaired patient may more easily and accurately sense the two ends of the pain scale **(70)** and understand the relative level of pain indicated on the pain scale.

The patient's pain scale **(70)** illustrated in this embodiment further comprises a dimension that varies in size along the length of the patient's pain scale **(70)** to further enhance the visually-impaired patient's ability to sense the relative position along the pain scale between the two ends so that the patient may more accurately indicate the relative level of pain on the pain scale **(70)**. More particularly, the patient's pain scale **(70)** in this embodiment is wedge shaped with a narrow end **(74)**, representing the "no pain" end of the pain scale, and a wide end **(76)** representing the "maximum pain" end of the pain scale. In addition, two pain range markers **(72, 78)** are provided to label the two ends **(74, 76)** of the patient's pain scale **(70)**. The pain range marker **(72)** at the no pain end of the scale may be a short dash and the pain range marker **(78)** at the maximum pain end of the scale may be a longer dash. These pain range markers **(72, 78)** are also provided in relief, serving as additional means for the visually-impaired patient to distinguish the two ends of the scale. It will be obvious to one of ordinary skill in the art that these pain range markers may be provided in any number of shapes other than dashes as long as the visually-impaired patient can distinguish them by touch. For example, the pain range markers may be two circular disk-like forms having two different diameters.

The patient's side **(14)** also may be labeled with texts such as "No Pain" **(80)** and "Maximum Pain Conceivable" **(82)** or other similar words near the narrow end **(74)** and the wide end **(76)** respectively. The texts and markings will enable the analog scale **(10)** to be more easily used by both visually-abled as well as visually-impaired patients.

In alternative embodiments, a plain straight-line patient's pain scale **(40)** similar to the patient's pain scales found in prior art VAS devices may be provided in addition to the primary patient's pain scale **(70)**. This straight-line patient's pain scale **(40)** may be provided to accommodate those visually-abled patients who might be more accustomed to using the traditional prior art VAS devices.

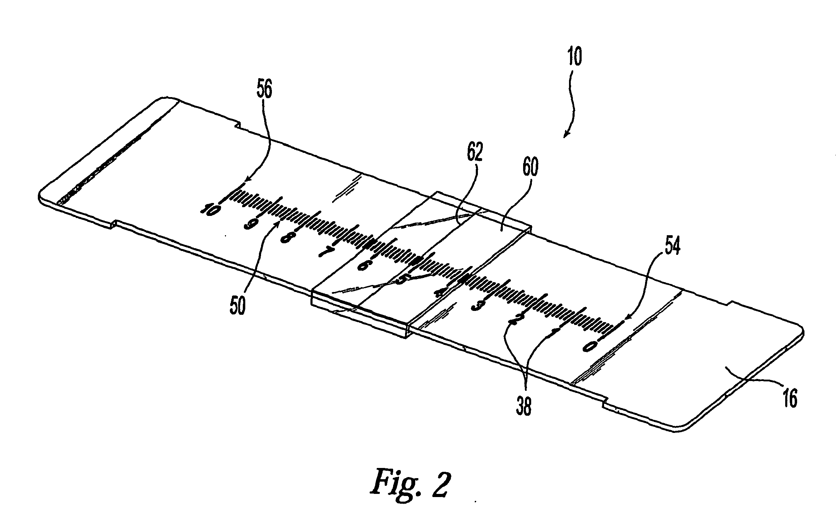

**Figure 2** illustrates the healthcare provider's side **(16)** of the analog scale **(10)**. The healthcare provider's side **(16)** displays a provider's pain scale **(50)** whose length corresponds with the patient's pain scale **(70)**. The provider's pain scale is a graduated scale and marked with numerical pain descriptors **(38)** ranging from 0 to 10 at uniform discrete intervals between a first end **(54)** and a second end **(56)**. The first end **(54)** of the provider's pain scale **(50)** corresponds to the narrow end **(74)** of the patient's pain scale **(70)** on the patient's side **(14)** and is marked with the numerical pain descriptor "0". The second end **(56)** of the provider's pain scale **(50)** corresponds to the wider end **(76)** of the patient's pain scale **(70)** on the patient's side **(14)** and is marked with the numerical pain descriptor "10"**.** The provider's side of the pain scale may also illustrate appropriate text pain descriptors corresponding to the graduated scale numbers.

The analog scale **(10)** is also provided with a slider **(60)** that is mounted on the analog scale **(10)** in such manner that the slider **(60)** is slidable between the two ends of the analog scale **(10).** As illustrated in **Figure 2****,** the slider **(60)** overlays the provider's pain scale **(50)** and is made from a transparent or translucent material so that the provider's pain scale, which illustrates numerical and/or text pain descriptors, can be seen through the slider. The slider **(60)** may also be made from an opaque material and include an opening so that the provider's pain scale descriptors may be seen through the slider.

The slider **(60)** is configured with a first arm portion **(64)** and a second arm portion (66) which wrap around the analog scale **(10)** to the patient's side **(14).** In the embodiment illustrated in **Figures 1** and **2****,** the two arms of the slider **(60)** do not meet on the patient's side **(14)**, but leave a gap or a channel between the two arm portions **(64, 66),** so that the arm portions flank the patient's pain scale **(70).**

The slider **(60)** may be provided with one or more indicators **(62),** which, in this embodiment, is provided in the form of a hairline that extends from the provider's side to the two arms on the patient's side. The patient uses the indicator **(62)** on the two arm portions **(64, 66)** to move the slider **(60)** to a point along the patient's pain scale **(70)** which corresponds to the particular pain level which the patient is experiencing. When positioned by the patient, the indicator **(62)** will illustrate, on the healthcare provider's side, the numerical and/or text representation of the pain level indicated by the patient.

In the embodiment of the invention shown in **Figures 1** and **2**, the indicator **(62)** is illustrated as a hairline. In other embodiments of the invention, the indicator **(62)** may be comprised of one or more markings of any shape that are appropriate for the purpose. For example, an arrow, a vertical dash, or a dot are all appropriate forms for the indicator **(62).** An important feature is that the relative positions of the indicators on the patient's side and the provider's side must be properly configured so that they point to the correct corresponding positions on the patient's pain scale and the provider's pain scale.

For a visually-abled patient to use a pain scale as illustrated in **Figure 1**, the patient may simply slide the slider **(60)** to an appropriate position along the patient's pain scale **(70)** so that the indicator **(62)** is pointing to a desired point along the pain scale. But for a visually-impaired patient, the indicator must also be recognizable by touch. To enable a visually-impaired patient to feel the indicator, the two arm portions **(64, 66)** of the slider **(60)** illustrated in **Figure 1** are provided with center points **(68)** at the two ends of the indicator **(62).** The center points **(68)** protrude from the two arm portions **(64, 66).** Thus, by placing a finger between the two arms **(64, 66)** a visually-impaired patient would be able to feel the center points **(68)** along with the patient's pain scale **(70)** so that the patient can properly position the slider along the pain scale.

Alternatively, the center points **(68)** may be notches cut into the slider rather than the protruding structures as shown in **Figure 1****.** As a further alternative to the center point structure, the indicator **(62)** may be raised on or carved into the slider's surface. These and other alternatives may be used in any combination to achieve the desired sense perception by visually-impaired patients.

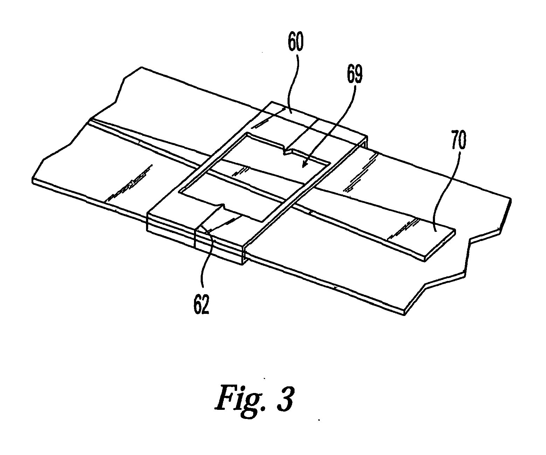

In another embodiment, slider **(60)** may be configured to have a continuous one-piece arm on the patient's side, rather than two arm portions, and further provided with an opening (69) to enable the patient to touch the patient's pain scale **(70)** through the opening. Such a slider is illustrated in **Figure 3****.**

Although the patient's pain scale **(70)** and the two pain range markers **(72, 78)** are illustrated in particular form as shown in **Figure 1****,** it will be obvious to one of ordinary skill in the art that these features may be provided in many other appropriate forms which may be interpreted by touching the scale as well as by viewing the scale. For example, the patient's pain scale **(70)** may be provided in a shape other than the wedge shape illustrated in **Figure 1****.** The patient's pain scale may be a straight horizontal bar as long as the visually-impaired person knows which ends represent the "no pain" and the "maximum pain conceivable" ends of the pain scale.

Also included within the scope of the present invention are the variety of ways in which the patient's pain scale may be configured to exhibit its three dimensional structure that may be recognized and interpreted by visually-impaired patients. For example, the patient's pain scale **(70)** and the two pain range markers **(72, 78)** may be provided in negative relief (i.e. provided as indentations on the surface of the analog scale **(10))**; the patient's pain scale may vary in thickness from one end to the other (i.e., thinnest at the "no pain" end **(74)** and thickest at the "maximum pain" end **(76))**; or the silhouettes of the patient's pain scale and the pain range markers may be cut into the analog scale as holes. Thus, the height and/or depth of the bar on the patient's pain scale may be raised in any number of way which allow for interpretation of the indicated relative level of pain. Also, braille may be provided which represents numerical and/or text representations of the indicated relative level of pain.

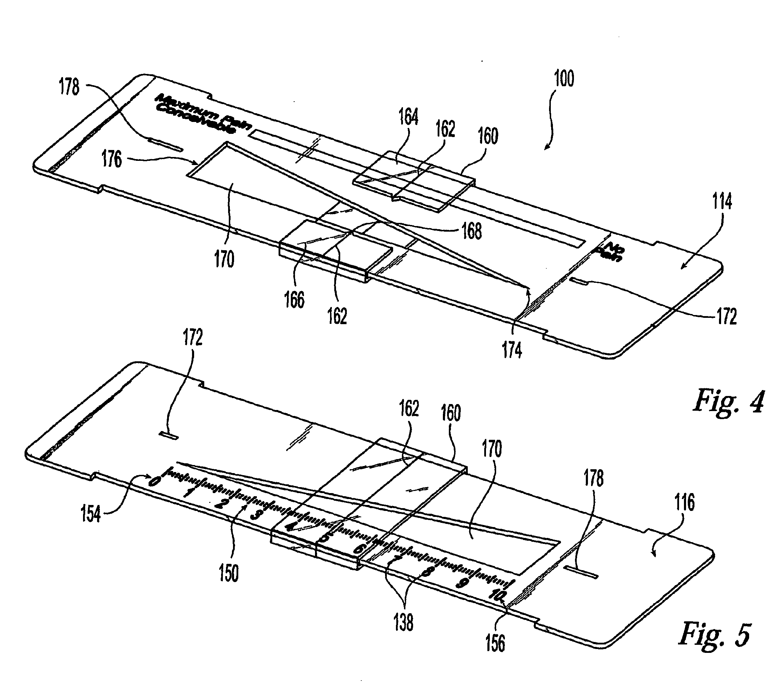

An embodiment of the invention according to the last example listed above is illustrated in **Figures 4** and **5. Figure 4** illustrates the patient's side **(114)** of analog scale **(100).** Patient's pain scale **(170)** and two pain range markers **(172,178)** are holes cut out of the device. Slider **(160)** has two arm portions **(164,166)** with a gap between them so that the visually-impaired patient can place a finger between the two arm portions and touch the patient's pain scale **(170).** The slider **(160)** has an indicator **(162)** and center points **(168)** similar to the slider **(60)** of the analog scale **(10)** illustrated in **Figures 1** and **2****.**

**Figure 5** illustrates the provider's side **(116)** of the analog scale **(100).** As in the embodiment illustrated in **Figure 2****,** provider's pain scale **(150)** is graduated and marked with numerical pain descriptors **(138).** Unlike the other embodiments discussed above, the back sides of the patient's pain scale **(170)** and the pain range markers **(172,178)** can be seen through the provider's side **(116).**

In another embodiment, the analog scale may have a circular structure where the patient's pain scale **(70)** and the provider's pain scale **(50)** are arranged around the perimeter of the circular device. The pain scales may span the entire perimeter or an arc of the circle and the slider would then be configured and adapted to rotate around the circular device.

The various embodiments of the patient's pain scale described herein are not meant to be exhaustive but merely samples of the patient's pain scale that are within the scope of the present invention. Many other configurations for the pain scale will be obvious to one of ordinary skill in the art that will provide the necessary three dimensional structure for the pain scale so that the visually-impaired patient can feel the scale.

In use, the visually-impaired patient is presented with the patient's side **(14,114)** of the analog scale **(10,100)** of the present invention. The patient positions his or her finger in an opening or a gap in the slider on the patient's side so that he or she can touch the patient's pain scale **(70,170)** while sliding the slider **(60,160)** along the patient's pain scale **(70,170).** The patient may move his or her finger several times back and forth along the patient's pain scale **(70,170)** between the two ends in order to interpret and understand the patient's pain scale **(70,170).** The patient then indicates the level of pain being experienced by positioning the slider **(60,160)** at an appropriate point along the patient's pain scale **(70,170).** The indicator **(62,162)** on the provider's side of the slider **(60,160)** will be pointing to a point along the numerical pain scale of 0 to 10 which corresponds to the pain level that was qualitatively indicated by the patient's positioning of the indicator **(62)** on the patient's pain scale. The healthcare provider can use the numerical and/or text representation of the indicated pain level to assist in the diagnosis and treatment of the patient's pain.

The illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated. It is intended that the scope of the invention be defined by the following claims and their equivalents.

## Claims

1. An analog scale (10) for diagnosing a level of pain experienced by a person comprising;
a panel having a patient's side and a provider's side;
a patient's pain scale (70) having a first end (74) and a second end provided (76) on said patient's side of said panel, said patient's pain scale having a three dimensional structure, said first end (74) representing the lowest level of pain (80) and said second end (76) representing the highest level of pain (82);
a provider's pain scale (50) on said provider's side of the panel having said first end (54) and said second end (56), said first end (54) of said provider's pain scale (50) corresponding to the first end (74) of the patient's pain scale (70) and said second end (56) of the provider's pain scale (50) corresponding to the second end (76) of the patient's pain scale (70);
a slider (60) having at least one indicator (68) and slidably mounted on the panel and movable between the first end and second ends, of the patients pain scale (70) and provider's pain scale (50).
**characterised by**;
the panel having two sides (14 & 16), wherein one side is the patient's side (14) and the other side is a provider's side (16), wherein the patient's side and the provider's side are opposing surfaces of the panel.

2. An analog scale (10) according to claim 1, wherein said patient's pain scale (70) comprises a relief (70) raised on the patient's side of the panel.

3. An analog scale according to claim 1, wherein said patient's pain scale comprises a negative relief on the patient's side of the panel.

4. An analog scale (10) according to claim 1, wherein said patient's pain scale comprises a hole in the panel.

5. An analog scale (10) according to claim 1, wherein said patient's pain scale (70) further comprises at least one dimension that varies in size along its length.

6. An analog scale (10) according to claim 5, wherein said patient's pain scale (70) varies in thickness along its length.

7. An analog scale (10) according to claim 5, wherein said patient's pain scale (70) is wedge shaped having a narrow end and a wide end, said narrow end being the first end (74) of the patient's pain scale and said wide end being the second end (76) of the patient's pain scale (70).

8. An analog scale (10) according to claim 1, wherein said slider (60) has two arm portions (64 and 66) flanking the patients pain scale (70) on the patient's side of the analog scale (10) forming a gap between said two arm portions (64 and 66) sufficiently large for a patient to place a finger tip into said gap and touch the patient's pain scale (70); and
at least one of the said arm portions (64 and 66) having an indicator (62).

9. An analog scale (10) according to claim 8, wherein said slider (60) further comprises at least one center point (68) associated with said indicator (62).

10. An analog scale (10) according to claim 1, wherein said slider (60) has an opening on the patient's side of the analog scale sufficiently large for a patient to place a finger tip into said opening and touch the patient's pain scale.

11. An analog scale (10) according to claim 10, wherein said slider (60) further comprises at least one center point (68) associated with said indicator (62).

12. An analog scale (10) according to claim 1, wherein said slider (60) has an indicator (62) that is configured as a three dimensional structure (70) at least on the patient's side of the analog scale (10).

13. An analog scale (10) according to claim 1, further comprising a straight-line patient's pain scale (40) provided on the patient's side.

14. An analog scale (10) according to claim 1, further comprising a first and a second pain range marker (72 and 78) provided on the patient's side, each pain range marker distinguishable from the other, said first pain range marker (72) located near the first end of the patient's pain scale (70) and said second pain range marker (78) is located near the second end of the patient's pain scale (70), wherein the first and the second pain range markers have three dimensional structures.

15. An analog scale (10) according to claim 14, wherein the two pain range markers (72 and 78) comprise dashes of different lengths.

16. An analog scale (10) according to claim 1, wherein said provider's pain scale (50) comprises a graduated scale marked with numerical pain descriptors (38).

17. An analog scale (10) according to claim 1, wherein said pateint's pain scale (70) is provided in a relief raised on the patient's side of the panel (14); and
said provider's pain scale (50) comprising a graduated scale marked with pain descriptors (38); and
said slider (60) has two arm portions (64 and 66) flanking the patient's pain scale (70) on the patient's side (14) of the analog scale (10) forming a gap between said two arm portions (64 and 66) sufficiently large for a patient to place a finger tip into said gap and touch the patient's pain scale (70).

18. An analog scale (10) according to claim 17, wherein said patient's pain scale (70) is wedge shaped having a narrow end (74) and a wide end (76), said narrow end (74) being the first end (74) of the patient's pain scale (70) and said wide end (76) being the second end (76) of the patient's pain scale (70).

19. An analog scale (10) according claim 17, wherein said pain descriptors comprise numerical pain descriptors (38).

## Patentansprüche

1. Analoge Skala (10) für das Diagnostizieren eines von einer Person empfundenen Schmerzniveaus, die aufweist:
eine Tafel mit einer Patientenseite und einer Versorgungspersonalseite;
eine Schmerzskala (70) für den Patienten mit einem ersten Ende (74) und einem zweiten Ende (76), das auf der Patientenseite der Tafel vorhanden ist, wobei die Schmerzskala für den Patienten einen dreidimensionalen Aufbau aufweist, wobei das erste Ende (74) das niedrigste Schmerzniveau (80) und das zweite Ende (76) das höchste Schmerzniveau (82) verkörpert;
eine Schmerzskala (50) für das Versorgungspersonal auf der Versorgungspersonalseite der Tafel, die das erste Ende (54) und das zweite Ende (56) aufweist, wobei das erste Ende (54) der Schmerzskala (50) für das Versorgungspersonal dem ersten Ende (74) der Schmerzskala (70) für den Patienten entspricht, und wobei das zweite Ende (56) der Schmerzskala (50) für das Versorgungspersonal dem zweiten Ende (76) der Schmerzskala (70) für den Patienten entspricht;
einen Schieber (60) mit mindestens einer Anzeigeeinrichtung (68) und verschiebbar auf der Tafel montiert und zwischen dem ersten Ende und den zweiten Enden der Schmerzskala (70) für den Patienten und der Schmerzskala (50) für das Versorgungspersonal beweglich,
**dadurch gekennzeichnet, dass**:
die Tafel zwei Seiten (14 & 16) aufweist, wobei eine Seite die Patientenseite (14) und die andere Seite die Versorgungspersonalseite (16) ist, wobei die Patientenseite und die Versorgungspersonalseite gegenüberliegende Flächen der Tafel sind.

2. Analoge Skala (10) nach Anspruch 1, bei der die Schmerzskala (70) für den Patienten eine Reliefform (70) aufweist, die auf der Patientenseite der Tafel erhöht ist.

3. Analoge Skala nach Anspruch 1, bei der die Schmerzskala für den Patienten eine negative Reliefform auf der Patientenseite der Tafel aufweist.

4. Analoge Skala (10) nach Anspruch 1, bei der die Schmerzskala für den Patienten ein Loch in der Tafel aufweist.

5. Analoge Skala (10) nach Anspruch 1, bei der die Schmerzskala (70) für den Patienten außerdem mindestens eine Dimension aufweist, die in der Größe entlang ihrer Länge variiert.

6. Analoge Skala (10) nach Anspruch 5, bei der die Schmerzskala (70) für den Patienten in der Dicke entlang ihrer Länge variiert.

7. Analoge Skala (10) nach Anspruch 5, bei der die Schmerzskala (70) für den Patienten keilförmig mit einem schmalen Ende und einem breiten Ende ist, wobei das schmale Ende das erste Ende (74) der Schmerzskala für den Patienten und das breite Ende das zweite Ende (76) der Schmerzskala (70) für den Patienten sind.

8. Analoge Skala (10) nach Anspruch 1, bei der der Schieber (60) zwei Armabschnitte (64 und 66) aufweist, die die Schmerzskala (70) für den Patienten auf der Patientenseite der analogen Skala (10) flankieren, wobei ein Spalt zwischen den zwei Armabschnitten (64 und 66) gebildet wird, der ausreichend groß ist, damit ein Patient eine Fingerspitze im Spalt anordnen und die Schmerzskala (70) für den Patienten berühren kann; und
mindestens einer der Armabschnitte (64 und 66) eine Anzeigeeinrichtung (62) aufweisen.

9. Analoge Skala (10) nach Anspruch 8, bei der der Schieber (60) außerdem mindestens einen Mittelpunkt (68) in Verbindung mit der Anzeigeeinrichtung (62) aufweist.

10. Analoge Skala (10) nach Anspruch 1, bei der der Schieber (60) eine Öffnung auf der Patientenseite der analogen Skala aufweist, die ausreichend groß ist, damit ein Patient eine Fingerspitze in der Öffnung anordnen und die Schmerzskala für den Patienten berühren kann.

11. Analoge Skala (10) nach Anspruch 10, bei der der Schieber (60) außerdem mindestens einen Mittelpunkt (68) in Verbindung mit der Anzeigeeinrichtung (62) aufweist.

12. Analoge Skala (10) nach Anspruch 1, bei der der Schieber (60) eine Anzeigeeinrichtung (62) aufweist, die als ein dreidimensionaler Aufbau (70) mindestens auf der Patientenseite der analogen Skala (10) ausgebildet ist.

13. Analoge Skala (10) nach Anspruch 1, die außerdem eine geradlinige Schmerzskala (40) für den Patienten aufweist, die auf der Patientenseite vorhanden ist.

14. Analoge Skala (10) nach Anspruch 1, die außerdem eine erste und eine zweite Schmerzbereichsmarkierung (72 und 78) aufweist, die auf der Patientenseite vorhanden sind, wobei jede Schmerzbereichsmarkierung von der anderen unterscheidbar ist, wobei die erste Schmerzbereichsmarkierung (72) nahe des ersten Endes der Schmerzskala (70) für den Patienten und die zweite Schmerzbereichsmarkierung (78) nahe des zweiten Endes der Schmerzskala (70) für den Patienten angeordnet sind, wobei die erste und die zweite Schmerzbereichsmarkierung einen dreidimensionalen Aufbau aufweisen.

15. Analoge Skala (10) nach Anspruch 14, bei der die zwei Schmerzbereichsmarkierungen (72 und 78) Striche von unterschiedlichen Längen aufweisen.

16. Analoge Skala (10) nach Anspruch 1, bei der die Schmerzskala (50) für das Versorgungspersonal eine Messskala aufweist, die mit numerischen Schmerzdeskriptoren (38) markiert ist.

17. Analoge Skala (10) nach Anspruch 1, bei der die Schmerzskala (70) für den Patienten in einer Reliefform vorhanden ist, die auf der Patientenseite (14) der Tafel erhöht ist;
und wobei die Schmerzskala (50) für das Versorgungspersonal eine Messskala aufweist, die mit Schmerzdeskriptoren (38) markiert ist; und
wobei der Schieber (60) zwei Armabschnitte (64 und 66) aufweist, die die Schmerzskala (70) für den Patienten auf der Patientenseite (14) der analogen Skala (10) flankieren, wobei ein Spalt zwischen den zwei Armabschnitten (64 und 66) gebildet wird, der ausreichend groß ist, damit ein Patient eine Fingerspitze im Spalt anordnen und die Schmerzskala (70) für den Patienten berühren kann.

18. Analoge Skala (10) nach Anspruch 17, bei der die Schmerzskala (70) für den Patienten keilförmig mit einem schmalen Ende (74) und einem breiten Ende (76) ist, wobei das schmale Ende (74) das erste Ende (74) der Schmerzskala (70) für den Patienten und das breite Ende (76) das zweite Ende (76) der Schmerzskala (70) für den Patienten sind.

19. Analoge Skala (10) nach Anspruch 17, bei der die Schmerzdeskriptoren numerische Schmerzdeskriptoren (38) aufweisen.

## Revendications

1. Echelle analogique (10) pour diagnostiquer un niveau de douleur ressentie par une personne, comprenant :
un panneau, comportant un côté patient et un côté fournisseur de soins;
une échelle de la douleur du patient (70), comportant une première extrémité (74) et une deuxième extrémité (76) agencées sur ledit côté du patient dudit panneau, ladite échelle de la douleur du patient ayant une structure tridimensionnelle, ladite première extrémité (74) représentant le niveau minimal de douleur (80) et ladite deuxième extrémité (76) représentant le niveau maximal de douleur (82) ;
une échelle de la douleur du fournisseur de soins (50) sur ledit côté du fournisseur de soins du panneau, comportant ladite première extrémité (54) et ladite deuxième extrémité (56), ladite première extrémité (54) de ladite échelle de la douleur du fournisseur de soins (50) correspondant à la première extrémité (74) de l'échelle de la douleur du patient (70) et ladite deuxième extrémité (56) de l'échelle de la douleur du fournisseur de soins (50) correspondant à la deuxième extrémité (76) de l'échelle de la douleur du patient (70) ;
un coulisseau (60), comportant au moins un indicateur (68), monté de manière coulissante sur le panneau et pouvant être déplacé entre la première et deuxième extrémité de l'échelle de la douleur du patient (70) et de l'échelle de la douleur du fournisseur de soins (50) ;
**caractérisée en ce que** :
le panneau comporte deux côtés (14, 16), un côté constituant le côté patient (14) et l'autre côté constituant le côté du fournisseur de soins (16), le côté du patient et le côté du fournisseur de soins étant constitués par des surfaces opposées du panneau.

2. Echelle analogique (10) selon la revendication 1, dans laquelle l'échelle de la douleur du patient (70) comprend un relief (70) surélevé sur le côté du patient du panneau.

3. Echelle analogique (10) selon la revendication 1, dans laquelle l'échelle de la douleur du patient comprend un relief négatif sur le côté du patient du panneau.

4. Echelle analogique (10) selon la revendication 1, dans laquelle ladite échelle de la douleur du patient comprend un trou dans le panneau.

5. Echelle analogique (10) selon la revendication 1, dans laquelle ladite échelle de la douleur du patient (70) comprend en outre au moins une dimension changeant de taille le long de sa longueur.

6. Echelle analogique (10) selon la revendication 5, dans laquelle ladite échelle de la douleur du patient (70) a une épaisseur variable le long de sa longueur.

7. Echelle analogique (10) selon la revendication 5, dans laquelle ladite échelle de la douleur du patient (70) a une forme en biseau, avec une extrémité étroite et une extrémité large, ladite extrémité étroite constituant la première extrémité (74) de l'échelle de la douleur du patient et ladite extrémité large constituant la deuxième extrémité (76) de l'échelle de la douleur du patient (70).

8. Echelle analogique (10) selon la revendication 1, dans laquelle ledit coulisseau (60) comporte deux parties de bras (64 et 65) flanquant l'échelle de la douleur du patient (70) sur le côté du patient de l'échelle analogique (10), établissant un espace entre lesdites deux parties de bras (64 et 66), suffisamment grand pour qu'un patient puisse y placer la pointe d'un doigt dans ledit espace et toucher l'échelle de la douleur du patient (70) ; et
au moins une desdites parties de bras (64 et 66) comportant un indicateur (62).

9. Echelle analogique (10) selon la revendication 8, dans laquelle le coulisseau (60) comprend en outre au moins un point central (68) associé audit indicateur (62).

10. Echelle analogique (10) selon la revendication 1, dans laquelle ledit coulisseau (60) comporte une ouverture sur le côté du patient de l'échelle analogique, suffisamment grande pour qu'un patient puisse placer la pointe d'un doigt dans ladite ouverture et toucher l'échelle de la douleur du patient.

11. Echelle analogique (10) selon la revendication 10, dans laquelle ledit coulisseau (60) comprend en outre au moins un point central (68) associé audit indicateur (62).

12. Echelle analogique (10) selon la revendication 1, dans laquelle ledit coulisseau (60) comporte un indicateur (62), configuré sous forme d'une structure tridimensionnelle (70), au moins sur le côté du patient de l'échelle analogique (10).

13. Echelle analogique (10) selon la revendication 1, comprenant en outre une échelle de la douleur du patient à ligne droite (40) agencée sur le côté du patient.

14. Echelle analogique (10) selon la revendication 1, comprenant en outre des premier et deuxième marqueurs de l'étendue de la douleur (72 et 78) agencés sur le côté du patient, chaque marqueur de l'étendue de la douleur pouvant être distingué de l'autre, ledit premier marqueur de l'étendue de la douleur (72) étant agencé près de la première extrémité de l'échelle de la douleur du patient (70) et ledit deuxième marqueur de la douleur (78) étant agencé près de la deuxième extrémité de l'échelle de la douleur du patient (70), les premier et deuxième marqueurs de l'étendue de la douleur ayant des structures tridimensionnelles.

15. Echelle analogique (10) selon la revendication 14, dans laquelle les deux marqueurs de l'étendue de la douleur (72 et 78) comprennent des traits de différentes longueurs.

16. Echelle analogique (10) selon la revendication 1, dans laquelle ladite échelle de la douleur du fournisseur de soins (50) comprend une échelle graduée, marquée par des descripteurs numériques de la douleur (38).

17. Echelle analogique (10) selon la revendication 1, dans laquelle ladite échelle de la douleur du patient (70) est agencée dans un relief surélevé sur le côté du patient du panneau (14) ; et
ladite échelle de la douleur du fournisseur de soins (50) comprenant une échelle graduée marquée par des descripteurs de la douleur (38) ; et
ledit coulisseau (60) comportant deux parties de bras (64 et 66), flanquant l'échelle de la douleur du patient (70) sur le côté du patient (14) de l'échelle analogique (10), établissant un espace entre lesdites deux parties de bras (64 et 66), suffisamment large pour qu'un patient puisse placer la pointe d'un doigt dans ledit espace et toucher l'échelle de la douleur du patient (70).

18. Echelle analogique (10) selon la revendication 17, dans laquelle ladite échelle de la douleur du patient (70) a une forme en biseau, comportant une extrémité étroite (74) et une extrémité large (76), ladite extrémité étroite (74) constituant la première extrémité (74) de l'échelle de la douleur du patient (70), et ladite extrémité large (76) constituant la deuxième extrémité (76) de l'échelle de la douleur du patient (70).

19. Echelle analogique (10) selon la revendication 17, dans laquelle lesdits descripteurs de la douleur comprennent des descripteurs numériques de la douleur (38).
